# EUROPEAN PATENT SPECIFICATION

(11) **EP 0 602 478 B1**
(45) Date of publication and mention of the grant of the patent: **31.07.1996**
(21) Application number: 93119528.3
(22) Date of filing: 03.12.1993
(51) Int. Cl.: C07H 19/067

(54) **Novel process for producing N4-acyl-5'-deoxy-5-fluorocytidine derivatives**
Verfahren zur Herstellung von N4-acyl-5'-desoxy-5-fluorocytidinderivate
Procédé pour la production des dérivés de N4-acyl-5'-déoxy-5-fluorocytidine

(30) Priority: 18.12.1992 EP 92121539
(43) Date of publication of application: 22.06.1994
(73) Proprietor: F. HOFFMANN-LA ROCHE AG, 4002 Basel (CH); FUJI KAGAKU KOGYO KABUSHIKI KAISHA, Nakaniikawa-gun, Toyama-ken (JP)
(72) Inventor: Kamiya, Takashi, Osaka-hu (JP); Ishiduka, Makoto, Toyama-shi, Toyama-ken (JP); Nakajima, Hiroshi, Toyama-shi, Toyama-ken (JP)
(74) Representative: Keller, Günter, Dr.

(56) References cited:
- EP-A- 0 316 704
- EP-A- 0 373 485

## Description

This invention relates to a novel process for producing N⁴-acyl-5'-deoxy-5-fluorocytidine derivatives which are useful as drugs. The novel process utilizes novel 5'-deoxy-5-fluoro-N⁴-2'-O,3'-O-triacylcytidine derivatives as intermediates.

N⁴-acyl-5'-deoxy-5-fluorocytidine derivatives represented by the formula set forth below have antitumor activity [*Japanese Journal of Cancer Research*, Vol. 81, PP. 188 ∼ 195 (1990)]: 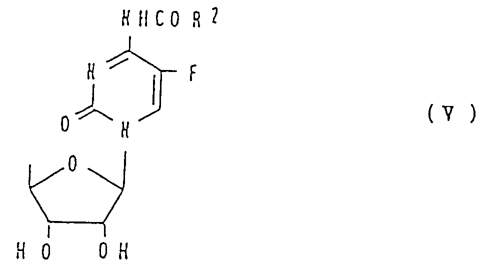 wherein R² is an alkyl radical, a cycloalkyl radical, an alkenyl radical, an aralkyl radical, an aryl radical or an alkoxy radical.

A process for producing said compounds starting from 5'-deoxy-5-fluorocytidine is described in Japanese Patent Application Kokai No. 153,696/1989.

In order to selectively introduce an acyl radical (R²CO) into an amino radical of this compound, this method involves the steps of (1) introducing a protective radical such as an isopropylidene radical, a silyl radical or the like into a hydroxy radical in the sugar part of this compound, (2) subsequently acylating the amino radical in the cytosine-base part and (3) finally eliminating the protective radical using an acid catalyst or the like. 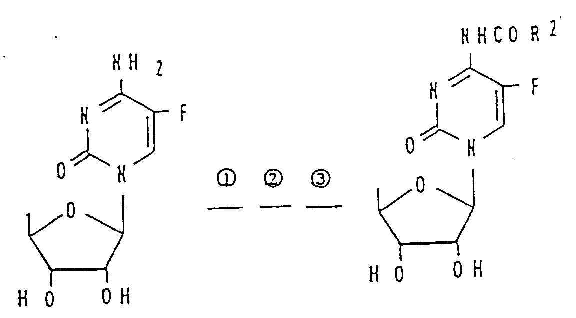 wherein R² is the same as above.

In the above method, a protective radical which is unnecessary in the molecular structure of the final compound, must be introduced and eliminated.

In addition, 5'-deoxy-5-fluorocytidine as a starting substance is produced, for example, from 5-fluorocytosine through 5-fluorocytidine [*Chem Pharm Bull*., Vol. 26, No. 10, P. 2,990 (1978)]. However, this method requires many steps (cf. Japanese Patent Publication No. 34,479/1983).

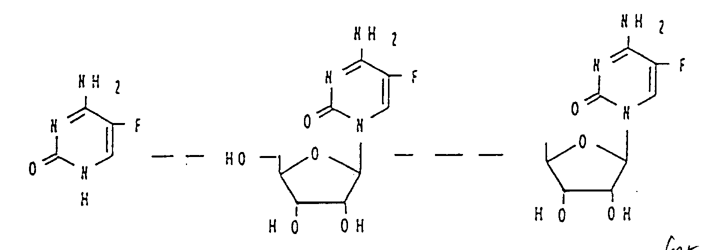

Thus, conventional production methods for N⁴-acyl-5'-deoxy-5-fluorocytidine derivatives involve steps in which protection of a hydroxy radical in the sugar part and/or an amino radical in the cytosine part with suitable protective radical(s) and elimination of said protective radical(s) after completion of the desired reactions must be carried out repeatedly, so that they are not easily performable on an industrial scale.

This invention purports to provide a novel production process by which the above N⁴-acyl-5'-deoxy-5-fluorocytidine derivatives of formula V can be produced very simply and easily. In the novel production process a novel compound represented by the following general formula (IV), 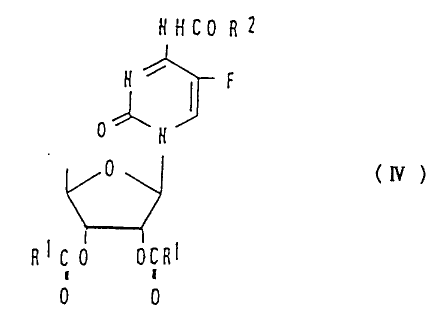 wherein R¹ is a lower alkyl radical, an aryl radical or an aryl radical which may have (a) substituent(s) and R² is an alkyl radical, a cycloalkyl radical, an alkenyl radical, an aralkyl radical, an aryl radical or an alkoxy radical, is used. The step of selectively eliminating the acyl radicals from its sugar part constitutes a characteristic feature which makes it possible to produce N⁴-acyl-5'-deoxy-5-fluorocytidine derivatives in few steps and in excellent yields as compared with conventional processes.

The above characteristic step of selective deacylation was unexpected from the conventional techniques with respect to yield and the purity of the product.

Generally, it was known that mainly an O-acyl radical is eliminated, when reacting N⁴,O-acylcytidine derivatives with an alkali. However, the cleavage of an N-acyl radical also takes place, so that complicated operations of separation and purification were required in order to obtain a compound from which only the O-acyl radical was eliminated with satisfactory selectivity [J. H. van Boom et al., *Nucleic Acids Research*, Vol. 4 (4), pp. 1'047 - 1'063 (1977)].

An N⁴⁻acyl radical is relatively easily cleaved from N⁴,O-acylcytidines. For example, from N⁴,2'-0,3'-0,5'-O-tetracylcytidines, it was known that an N-acyl radical alone can be eliminated selectively by merely heating the same in alcohol (cf. Japanese Patent Application Kokai No. 23.085/1977).

In addition, it was also known that when a 5-fluoro-N⁴,2'-0,3'-0,5'-O-tetracylcytidine derivative is treated with 0.5N-sodium methoxide in methanol at room temperature, all acyl radicals are eliminated to yield 5-fluorocytidine [*Chem*. *Pharm*. *Bull*., Vol. 26, No. 10, p. 2'990 (1978)].

The document EP-A-0 316 704 discloses a process for preparing N⁴-acyl-5'-deoxy-5-fluorocytidines with intermediary protection of the 2',3'-hydroxy groups of 5'-deoxy-5-fluorocytidine, detailing ether, acetal, ketal, carbonate and orthoester protecting groups. The document EP-A-0 373 485 discloses a process for the preparation of 2'-deoxy-2'-methylidene-N⁴-alkanoyl-cytidines wherein trimethylsilyl groups are used for intermediary protection of the 3',5'-hydroxy groups.

By the present invention, it was found that the acyl radical in the sugar part of 5'-deoxy-5-fluoro-N⁴,2'-0,3'-O-triacylcytidine derivative can be efficiently selectively eliminated, whereby N⁴-acyl-5'-deoxy-5-fluorocytidine derivatives can be obtained in few steps in excellent yields. These findings are unexpected from the above discussed conventional techniques.

The present invention provides a process for producing N⁴-acyl-5'-deoxy-5-fluorocytidine derivatives represented by the general formula (V), 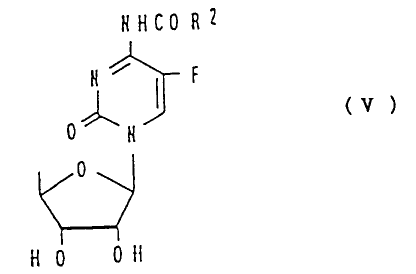 wherein R² is an alkyl radical, a cycloalkyl radical, an alkenyl radical, an aralkyl radical, an aryl radical or an alkoxy radical,
characterized by reacting 5-fluorocytosine with a compound represented by the general formula (II), 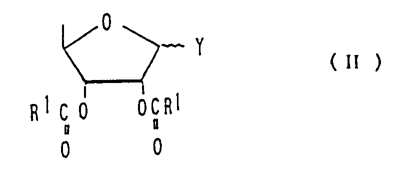 wherein R¹ is a lower alkyl radical, an aryl radical or an aryl radical which may have(a) substituent(s) and Y is a halogen atom, an acyloxy radical or an alkoxy radical,
to produce a compound represented by the general formula (III), 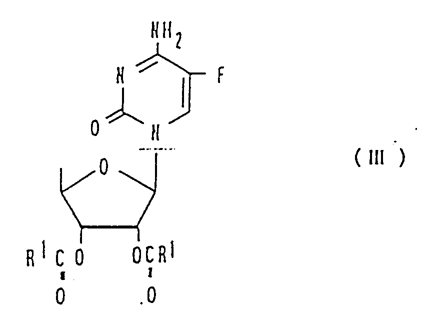 wherein R¹ is the same as defined above,
acylating the amino radical of this compound to produce a compound represented by the general formula (IV), 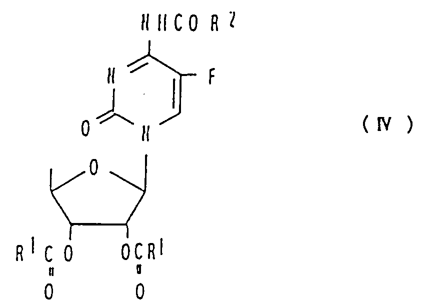 wherein R¹ and R² are the same as defined above, and selectively deacylating only the R¹CO radical of this compound. by treatment with a base in a solvent.

In addition, the present invention provides a process for producing N⁴-acyl-5'-deoxy-5-fluorocytidine derivatives represented by the general formula (V), 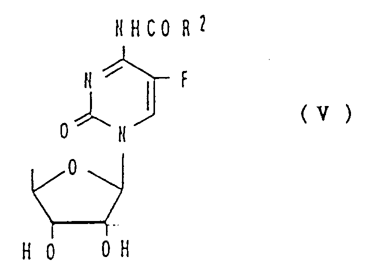 wherein R² is an alkyl radical, a cycloalkyl radical, an alkenyl radical, an aralkyl radical, an aryl radical or an alkoxy radical,
characterized by acylating the amino radical of 5-fluorocytosine to introduce an R²CO radical thereinto to produce a compound represented by the general formula (VI), 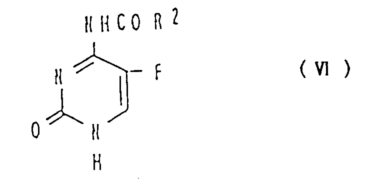 wherein R² is the same as defined above,
reacting this compound with a compound represented by the general formula (II), 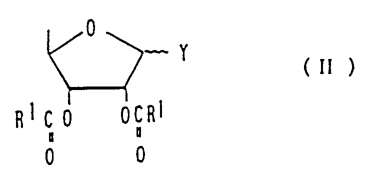 wherein R¹ is a lower alkyl radical, an aryl radical or an aryl radical which may have (a) substituent(s) and Y is a halogen atom, an acyloxy radical or an alkoxy radical,
to produce a compound represented by the general formula (IV), 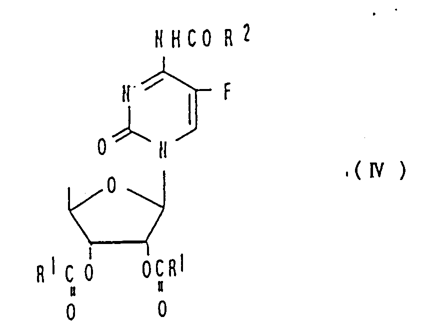 wherein R¹ and R² are the same as defined above, and selectively deacylating only R¹CO radicals of this compound. by treatment with a base in a solvent.

The above first process is represented by the following reaction formula:

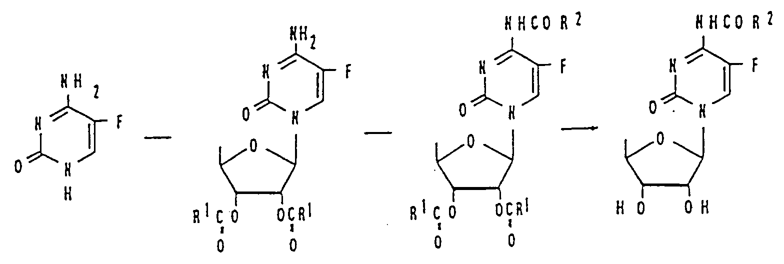

According to the first process of the present invention, N⁴-acyl-5'-deoxy-5-fluorocytidine derivatives can be produced using easily available 5-fluorocytosine as a starting material through very few steps, by simple operations, in excellent yields and at satisfactory purities.

Next, the reaction conditions will be described.

The compound of the general formula (III) can be obtained by reacting a silylation derivative of 5-fluorcytosine with a compound of the general formula (II) in a solvent in the presence of a catalyst suitably at a temperature of 0 ∼ 100°C.

The silylation derivative of 5-fluorocytosine can be obtained by reacting a silylating agent with 5-fluorocytosine according to conventional methods.

As the above silylating agent, hexamethyldisilazane, trimethylchlorosilane or the like can be used. The amount of a silylating agent to be used is preferably 0.5 ∼ 2 moles per mole of 5-fluorocytosine.

The reaction time for the above silylation, though it depends upon conditions such as the kind of starting materials, reaction temperature, the kind of base substances, kind of solvents, etc., is usually several hours.

As solvents for the above condensation reaction, there can be used for example, benzene, toluene, xylene, chloroform, methylene chloride, dichloroethane, carbon tetrachloride, 1,2-dichloropropane. 1,1,2,2-tetrachloroethane, acetonitrile, dioxane, tetrahydrofuran, etc.

In the compound of the general formula (II), R¹ denotes a lower alkyl radical, an aryl radical or an aryl radical which may have a substituent(s). Examples of the above lower alkyl radicals are methyl, ethyl, propyl isopropyl, butyl isobutyl or the like. Examples of aryl radicals are phenyl, methylphenyl nitrophenyl radical, or halogenophenyl.

Y means a halogen atom, e.g., fluorine, chlorine, bromine, iodine; an acyloxy radical, having 2-6 carbon atoms e.g., alkanoyloxy and benzoyloxy; benzoyloxy carrying on its ring a substituent such as methyl methoxy, nitro or a halogen or the like; and an alkoxy radical, e.g., methoxy, ethoxy or the like.

The compound of formula II is obtained from methyl(5-deoxy-2,3-O-isopropylidene)-D-ribofuranoside which can be obtained from D-ribose (see Japanese Patent Publication No. 40,239/1986). Examples thereof are 5-deoxy-1,2,3-tri-O-acyl-D-ribofuranoside, 5-deoxy-2,3-di-O-acyl-1-O-methyl-D-ribofuranoside, and 5-deoxy-2,3-di-O-acyl-1-halogeno-D-ribofuranoside.

Examples of the catalyst are, Lewis's acids such as tin tetrachloride, zinc chloride, boron fluoride, boron fluoride etherate, aluminum chloride, titanium tetrachloride, antimony chloride, ferric chloride, tin tetrabromide, zinc bromide, zirconium tetrachloride, silver nitrate, etc.; trifluoromethanesulfonic acid; trimethylsilyl trifluoromethanesulfonate; *ρ*-toluenesulfonic acid; and 2,4-dinitrophenol;

The compound of the general formula (III) can be obtained from the compound of the general formula (II) using the aforementioned solvents, catalysts, etc. suitably at a temperature of 0 ∼ 100°C.

The compound of the general formula (III ) can be obtained also by heating to melt silylated 5-fluorocytosine or 5-fluorocytosine and the compound of the general formula (II) in the presence of a catalyst, e.g., *ρ*-toluenesulfonic acid, 2,4-dinitrophenol or the like, without using a solvent.

The acylation of the compound of the general formula (III ) obtained according to the above process is carried out usually by reacting said compound with an activated carboxylic acid derivative represented by the general formula (VII),

R²CO-Z (VII)

wherein R² is the same as above and Z is a leaving radical,
in a solvent in the presence of a base substance at a suitable temperature.

Examples of the above activated carboxylic acid derivative are an acid halide, an active ester, an acid ester, an acid anhydride, and a mixed acid anhydride. Said activated carboxylic acid derivative can be produced according to conventional methods.

The amount of the compound of the general formula (VII) is suitably at least 1 mole per mole of the compound of the general formula (III).

The compound of the general formula (IV) can be produced also by reacting a compound of the general formula (III) and a carboxylic acid represented by the general formula

R²-COOH

wherein R² is the same as above,
with the addition of a condensing agent, e.g.. diethyl cyanophosphate, dicyclohexylcarbodiimide, *ρ*-toluenesulfonyl chloride, methanesulfonyl chloride or the like, if necessary, in the presence of a base according to conventional methods.

The amount of the condensing agent is suitably at least 1 mole per mole of the carboxylic acid.

The reaction time, though depending upon conditions such as kind of starting materials, reaction temperature, kind of bases, kind of solvents, etc., is usually several minutes to about 20 hours.

Examples of the base to be used for the above reaction, are organic bases such as triethylamine, tributylamine, pyridine, N,N-dimethylaminopyridine, lutidine, N-methylmorphorine, etc. and inorganic bases such as hydroxides, carbonates or alkoxides of alkali metals or alkaline earth metals, e.g., sodium hydroxide, sodium carbonate, sodium bicarbonate, sodium methoxide or their lithium salt, potassium salt, calcium salt, barium salt, etc.

In this specification, the term alkyl radical for R² means straight chain or branched chain alkyl radical carrying 1 ∼ 22 carbon atoms, for example, methyl, ethyl propyl, isopropyl butyl, isobutyl, *sec*-butyl *tert*-butyl, pentyl, isopentyl neopentyl; hexyl, isohexyl heptyl, octyl, nonyl, decyl, undecyl, dodecyl tridecyl, tetradecyl, pentadecyl, hexadecyl, heptadecyl, nonadecyl, etc.

The term cycloalkyl radical, means for example, cyclopropyl, cyclobutyl, cyclopentyl, cyclohexyl, adamantyl radical or the like.

The term alkenyl radical means an alkenyl carrying 2 ∼ 22 carbon atoms and having optional substituents(s), for example, allyl 1-propenyl butenyl, 3-methyl-2-butenyl, 1-methyl-2-propenyl, hexenyl decenyl, undecenyl, tridecenyl, pentadecenyl, heptadecenyl, heptadecadienyl, pentatridecatrienyl, nonadecenyl, nonadecadienyl, nonadecatetraenyl 2-phenylvinyl etc.

The term aralkyl radical means an aralkyl radical having optional substituent(s), for example, benzyl, 1-phenylethyl, methylbenzyl, fluorobenzyl, chlorobenzyl, methoxybenzyl, dimethoxybenzyl, nitrobenzyl, phenethyl, picolyl, a 3-indolylmethyl or the like.

The term aryl radical means an aryl radical having optional substituent(s), for example, phenyl, tolyl, xylyl, mesityl, cumenyl, ethylphenyl, fluorophenyl, chlorophenyl, bromophenyl, iodophenyl, difluorophenyl, dichlorophenyl methoxyphenyl, dimethoxyphenyl trimethoxyphenyl ethoxyphenyl diethoxyphenyl, triethoxyphenyl propoxyphenyl, methylenedioxyphenyl (methylthio)phenyl, nitrophenyl, cyanophenyl acetylphenyl, carbamoylphenyl, methoxycabamoylphenyl, naphthyl, biphenylyl, thienyl, methyl thienyl, furyl, nitrofuryl, pyrrolyl a methylpyrrolyl imidazolyl pyrazolyl pyridyl methylpyridyl pyrazinyl or the like.

The term alkoxy radical means, for example, methoxy cal, ethoxy, propoxy, butoxy, pentyloxy, hexyloxy, octyloxy, nonyloxy, etc.

As solvents for the above acylation, polar or non-polar solvents such as acetonitrile, chloroform, dichloroethane, methylene chloride, nitromethane, toluene, dimethyl formamide, acetone, dimethyl acetamide, hexamethyl, phosphoramide, dimethyl sulfoxide, pyridine, lutidine, etc. can be used.

In the above production process, the compound of the general formula (IV) is obtained in satisfactory purity and in good yields by treating the reaction solution according to conventional methods and then recrystallizing the residue from an appropriate solvent. The compound is obtained in good purity so that it can be used in the next step without particular isolation.

As solvents for recrystallization, there can be used, for example, alcohols such as methanol, ethanol, isopropyl alcohol, etc.; ethers such as isopropyl ether, etc.; methyl acetate; ethyl acetate; etc.

By recrystallization, a highly purified compound can be obtained if necessary.

Next, the reaction for producing N⁴-acyl-5'-deoxy-5-fluorocytidine derivatives from the compound of the general formula (IV) obtained according to the above process will be described.

This reaction is represented by the following reaction formula: 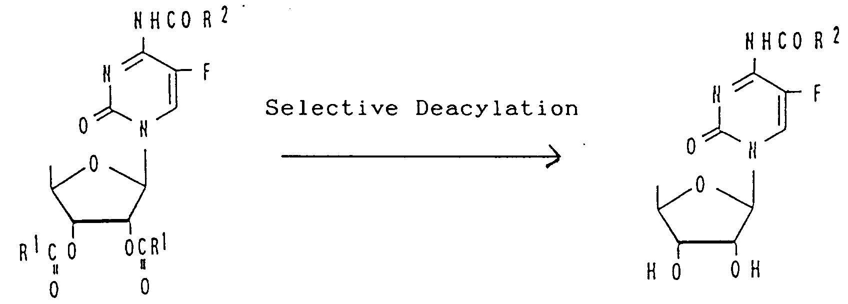 wherein R¹ and R² are the same as above.

In the above reaction the compound of the general formula (IV) is selectively deacylated in a solvent in the presence of a base.

The reaction temperature in this characteristic step is selected depending on the kind of materials, solvents, bases and their concentration, etc. Usually, it is at or below room temperature, preferably between 0°C and 30°C.

The reaction time, though depending upon conditions such as kind of starting materials, reaction temperature, kind of bases, kind of solvents, etc., is usually within the range of several minutes to about 20 hours.

The bases are dissolved in water or an organic solvent or a mixed solution of water and an organic solvent.

Both inorganic bases and organic bases can be used.

As an inorganic base, hydroxides, carbonates or alkoxide of alkali metal or alkaline earth metals such as sodium hydroxide, sodium carbonate, sodium bicarbonate, sodium methoxide, or their lithium salt, potassium salt, calcium salt, barium salt, etc. can be used. As an organic base, ammonia, triethylamine, DBU, tetramethylammonium hydroxide, strongly basic anion exchange resins (OH⁻ type), etc. can be used.

These bases can be used at any suitable concentration. However, it is usually preferred to used the same as a 0.4 ∼ 2N solution.

The amount of the base, though depending upon kind and combination of solvents to be used, is preferably within the range of 1 to 4 mole equivalents of the compound of the general formula (IV).

The solvents to be used are polar or non-polar solvents, for example, water; alcohols such as methanol, ethanol, propanol, bintanol, isopropanol; ethers such as tetrahydrofuran, dioxane; acetone; acid amides such as dimethyl formamide, carbon halogenides such as methylene chloride, chloroform, etc.; aromatic hydrocarbons such as toluene, xylene, etc. These can be used alone or in combination.

In case of using heterogeneous system, e.g. water and methylene chloride, etc., the desired product can be obtained in satisfactory yields.

In addition, when heterogeneous solvent systems are used, the reaction may be carried out with the addition of a phase transfer catalyst.

After the completion of reaction, the compound of the general formula (V) is obtained according to conventional separation and purification methods.

Industrially it is advantageous to use inorganic bases which are inexpensive.

As described above, the selective deacylation step which is characteristic of the present production process has an industrially very significant advantage because the compound of the formula (V) can be produced from the compound of the general formula (IV) by using an inexpensive base, by simple procedures, in good purity and in satisfactory yields.

The aforementioned novel compounds represented by the general formula (IV) can also be produced from 5-fluorocytosine by acylating 5-fluorocytosine and then reacting the obtained compound of the general formula (VI) 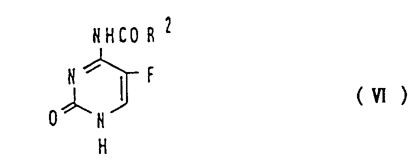 wherein R² is the same as above,
i.e. N⁴-acyl- 5-fluorocytosine derivative with the compound of the general formula (II).

Accordingly, the second process as describe above is an object of the present invention.

This reaction is carried out by reacting 5-fluorocytosine with the aforementioned above compound of the general formula (VII) in a solvent at a suitable temperature between room temperature to reflux temperature according to conventional methods.

The reaction time, though depending upon conditions such as kind of starting materials, reaction temperature, kind of bases, kind of solvents, etc. is usually several ten minutes to several hours.

As solvents, those used in the acylation in the afore-mentioned first process are used.

The amount of acylating agent is suitably at least 1 mole per mole of 5-fluorocytosine.

The compound of the general formula (IV) can be obtained by silylating the compound of the general formula (VI) obtained according to the above production process. i.e. by using the aforementioned silylating agent and then reacting the resulting compound with the compound of the general formula (II) in a solvent or in the absence of a solvent in the presence of a catalyst.

The reaction of the compound of the general formula (VI) or a silylation derivative of the compound of the general formula (VI) with the compound of the general formula (II) can be carried out under the same conditions as the aforementioned conditions for reacting 5-fluorocytosine with the compound of the general formula (II).

The amount of the silylating agent to be used is preferably 0.5 ∼ 2 moles per mole of the compound of the general formula (VI).

The reaction is usually carried out at or below room temperature. If necessary, ice-cooling may be applied.

The reaction time, though depending upon conditions such as kind of starting materials, reaction temperature, kind of solvents, etc., is usually several hours.

The production process of a compound represented by the above formula (IV) is represented by the following reaction formula: 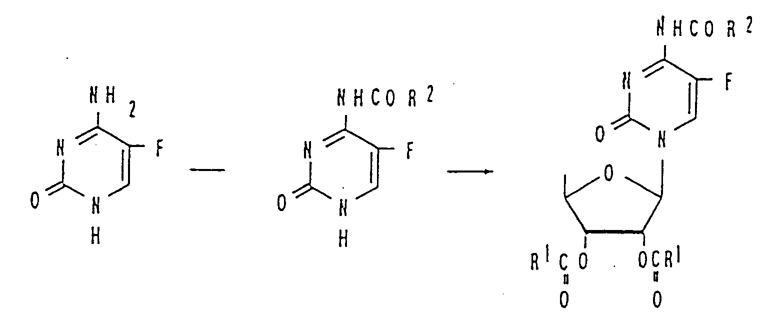 wherein R¹ and R² are the same as above.

The compound of the general formula (IV) can be obtained also by reacting the compound of the general formula (VI) with the compound of the general formula (II) in or without a solvent in the presence of a catalyst in the same manner as aforementioned.

The compounds represented by the general formula (IV) are novel compounds.

Hereinafter, typical compounds of formula IV will be exemplified.
5'-deoxy-2',3'-di-O-acetyl-5-fluoro-N⁴-palmitoylcytidine,
5'-deoxy-2',3'-di-O-acetyl-5-fluoro-N⁴-octyloxycarbonylcytidine,
5'-deoxy-2',3'-di-O-acetyl-5-fluoro-N⁴-(3-methylbenzoyl)cytidine,
5'-deoxy-2',3'-di-O-acetyl-5-fluoro-N⁴-(3,4,5-trimethoxybenzoyl)cytidine,
5'-deoxy-2',3'-di-O-acetyl-5-fluoro-N⁴-(2-methoxybenzoyl)cytidine,
N⁴-(4-chlorobenzoyl)-5'-deoxy-2',3'-di-O-acetyl-5-fluorocytidine,
5'-deoxy-2',3'-di-O-acetyl-5-fluoro-N⁴-(4-nitrobenzoyl)cytidine,
5'-deoxy-2',3'-di-O-acetyl-5-fluoro-N⁴-(2-furoyl)cytidine,
5'-deoxy-2',3'-di-O-acetyl-5-fluoro-N⁴-(nicotinoyl)cytidine,
5'-deoxy-2',3'-di-O-acetyl-5-fluoro-N⁴-(2-thenoyl)cytidine,
N⁴-crotonoyl-5'-deoxy-2',3'-di-O-acetyl-5-fluorocytidine,
N⁴-cyclohexanecarbonyl-5'-deoxy-2',3'-di-O-acetyl-5-fluorocytidine,
5'-deoxy-2',3'-di-O-acety-5-fluoro-N⁴-(phenylacetyl)cytidine, and,
5'-deoxy-2',3'-di-O-4-toluoyl-5-fluoro-N⁴-(3,4,5-trimethoxybenzoyl)cytidine.

Hereinafter, the present process will be described specifically, referring to examples.

### Examples

### Example 1

25.8g of 5-fluorocytosine was suspended in 103mℓ of toluene and 32.3g of hexamethyldisilazane. The mixture was heated to react at 110°C for 3 hours. After concentrating the reaction solution under reduced pressure, 330mℓ of methylene chloride and 59.3g of 5-deoxy-1,2,3-tri-O-acetyl-β-D-ribofuranoside were added to the residue. Then, 62.5g of anhydrous stannic chloride was added dropwise thereto over a period of 10 minutes while ice-cooling. After stirring the mixture at room temperature for additional 2 hours, 101g of sodium bicarbonate was added, and followed by dropwise addition of 35mℓ of water over a period of 20 minutes. After stirring the resulting mixture at room temperature for 3 hours, insoluble material was filtered off and the filtrate was washed with 100mℓ of 4% sodium bicarbonate solution. After removal of the solvent under reduced pressure, the residue was recrystallized from 180mℓ of isopropanol to obtain 49.9 g crystals (76%) of 5'-deoxy-2',3'-di-O-acetyl-5-fluorocytidine.

The melting point of a product after recrystallization of the above crystals from isopropanol was 191.5 ∼ 193.2°C.
UV Absorption Spectrum:
λₘₐₓ (H₂O) nm: 278 (ε=7,800), 239 (ε=8.800), 193 (ε=19,100)
Optical Rotation:
[α]_{D}(20°C): +86 (CHCℓ₃, C=1)
¹H-NMR (90MHz, CDCℓ₃):
1.45 (d, J=6.4Hz, 3H), 2.08 (s, 3H), 2.09 (s, 3H), 5.96 (dd, (J=4.4Hz, 1.5Hz), 1H), 7.38 (d, J=6.4Hz, 1H)

### Example 2

1.29g of 5-fluorocytosine was suspended in a solution of 16.5mℓ of methylene chloride and 3.4mℓ of acetonitrile. After adding 2.97g of 5-deoxy-1,2,3-tri-O-acetyl-β-D-ribofuranoside to the suspension, 3.91g of anhydrous stannic chloride was dropwise added within 5 minutes at room temperature. This solution was stirred at room temperature for 3 more hours, followed by subjecting the same after-treatment as in Example 1. After recrystallizing the residue from 7.4mℓ of ethanol, the crystals were filtered off to give 2.12g (64.4%) of 5'-deoxy-2',3'-di-O-acetyl-5-fluorocytidine.

The results of instrumental analysis of the obtained compound were identical with those of Example 1.

### Example 3

0.52g of 5-fluorocytosine was suspended in a solution of 2mℓ of toluene and 0.42g of hexamethyldisilazane, and the mixture was heated to 110°C for 3 hours. After concentrating the reaction mixture under reduced pressure, 6.6mℓ of methylene chloride and 1.19g of 5-deoxy-1,2,3-tri-O-acetyl-β-D-ribofuranoside were added to the residue. Then, 1.07g of trimethylsilyl trifluoromethanesulfonate was added at room temperature. After stirring the mixture at room temperature overnight, 13mℓ of saturated sodium bicarbonate was added. The mixture was stirred at room temperature for 30 minutes. After separation of the organic layer, the aqueous layer was extracted with 5mℓ of methylene chloride. The organic layers were combined, and washed with water. After removal of the solvent under reduced pressure. the residue was recrystallized from 6mℓ of isopropanol to give 0.69g (52.4%) of 5'-deoxy-2',3'-di-O-acetyl-5-fluorocytidine.

The results of instrumental analysis of the obtained compound were identical with those of Example 1.

### Example 4

38g of 5'-deoxy-2',3'-di-O-acetyl-5-fluorocytidine obtained according to the method of Example 1 was dissolved in 190mℓ of methylene chloride, followed by addition of 14.3g of pyridine. To this solution, 34.6g of 3,4,5-trimethoxybenzoyl chloride was added at room temperature. After stirring at room temperature overnight, the resulting solution was extracted with 152mℓ of methylene chloride and 76mℓ of water. The organic layer was separated and washed with 76mℓ of 4% sodium bicarbonate solution, and the solvent was distilled under reduced pressure. The residue was recrystallized from 620mℓ of methanol to obtain 58.2g (96.4%) of 5'-deoxy-2',3'-di-O-acetyl-5-fluoro-N⁴-(3,4,5-trimethoxybenzoyl)cytidine as a crystalline powder. The melting point of a product obtained by recrystallizing these crystals from ethylacetate was 130.8 ∼ 133.2°C.
UV Absorption Spectrum:
λₘₐₓ(H₂O) nm: 314 (ε=16,300), 255 (ε=11,100), 209 (ε=36,800)
Optical Rotation:
[α]_{D}(20°C): +45 (CHCℓ₃, C=1)
¹N-NMR (90MHz, CDCℓ₃):
1.48 (d, J=6.4Hz, 3H), 2.10 (s, 3H), 2.12 (s, 3H), 3.92 (s, 3H), 3.93 (s, 6H), 5.98 (dd, (J=4.9Hz, 1.0Hz), 1H), 7.48 (d, J=5.4Hz, 1H), 7.57 (s, 2H)

### Examples 5 ∼ 16

In the same manner as in Example 4, the compounds given in Tables 1, 2 and 3 were synthesized.

**Table 1**

| | Compounds Obtained | | | |
|---|---|---|---|---|
| | In Formula (IV) | | mp (°C) | ¹H-NMR (90MHz) δppm |
| | R¹ | R² | | |
| Example 5 | Me | CH₃-(CH₂)₁₄- | 78.6 ∼ 79.7 | (CDCℓ₃): 0.88 (bt, 3H), 1.25 (s, methylene), 1.47 (d, 3H), 2.10 (s, 3H), 2.11 (s, 3H), 4.28 (m, 1H), 4.99 (t, 1H), 5.35 (t, 1H), 5.95 (dd, 1H), 7.59 (d, 1H) |
| Example 6 | Me | CH₃-(CH₂)₇O- | Oily Substance | (CDCℓ₃): 0.88 (bt, 3H), 1.29 (bs, 10H), 1.47 (d, 3H), 1.71 (m, 2H), 2.10 (s, 3H), 2.11 (s, 3H), 4.17 (t, 2H), 5.01 (t, 1H), 5.29 (t, 1H), 5.96 (dd, 1H), 7.43 (d, 1H) |
| Example 7 | Me | 4-Chlorophenyl- | 128.4 ∼ 129.7 | (CDCℓ₃): 1.49 (d, 3H), 2.10 (s, 3H), 2.13 (s, 3H), 4.26 (m, 1H), 5.03 (t, 1H), 5.31 (t, 1H), 5.98 (dd, 1H), 7.42 (d, 2H), 7.50 (d, 1H), 8.24 (d, 2H) |
| Example 8 | Me | 2-Methoxyphenyl- | 139.3 ∼ 147.0 | (CDCℓ₃): 1.49 (d, 3H), 2.10 (s, 3H), 2.11 (s, 3H), 4.06 (s, 3H), 4.30 (m, 1H), 5.01 (t, 1H), 5.34 (t, 1H), 6.11 (dd, 1H), 7.69 (d, 1H), 8.25 (dd, 1H) |
| Example 9 | Me | 3-Methylphenyl- | 169.2 ∼ 170.4 | (CDCℓ₃): 1.48 (d, 3H), 2.10 (s, 3H), 2.11 (s, 3H), 2.41 (s, 3H), 4.25 (m, 1H), 5.03 (t, 1H), 5.33 (t, 1H), 5.98 (dd, 1H), 7.31 ∼ 7.38 (m, 2H), 7.52 (d, 1H), 8.07 (m, 2H) |

**Table 2**

| | Compounds Obtained | | | |
|---|---|---|---|---|
| | In Formula (IV) | | mp (°C) | ¹H-NMR (90MHz) δppm |
| | R¹ | R² | | |
| Example 10 | Me | 4-Nitrophenyl- | 186.8 ∼ 187.9 | (CDCℓ₃): 1.50 (d, 3H), 2.11 (s, 3H), 2.13 (s, 3H), 4.28 (m, 1H), 5.04 (t, 1H), 5.33 (t, 1H), 5.98 (dd, 1H), 7.57 (d, 1H), 8.21 ∼ 8.52 (m, 4H) |
| Example 11 | Me | 3-Pyridyl- | Amorphous powder | (CDCℓ₃): 1.49 (d, 3H), 2.11 (s, 3H), 2.13 (s, 3H), 4.28 (m, 1H), 5.04 (t, 1H), 5.33 (t, 1H), 5.98 (dd, 1H), 7.40 (m, 1H), 7.55 (d, 1H), 8.52 (m, 1H), 8.76 (dd, 1H), 9.47 (dd, 1H) |
| Example 12 | Me | 2-Furyl- | 139.6 ∼ 140.8 | (CDCℓ₃): 1.48 (d, 3H), 2.10 (s, 3H), 2.12 (s, 3H), 4.26 (m, 1H), 5.03 (t, 1H), 5.32 (t, 1H), 5.98 (dd, 1H), 6.55 (dd, 1H), 7.41 (dd, 1H), 7.52 (d, 1H), 7.64 (dd, 1H) |
| Example 13 | Me | 2-Thienyl- | 154.0 ∼ 154.8 | (CDCℓ₃): 1.48 (d, 3H), 2.10 (s, 3H), 2.12 (s, 3H), 4.25 (m, 1H), 5.03 (t, 1H), 5.31 (t, 1H), 5.97 (dd, 1H), 7.13 (dd, 1H), 7.48 (d, 1H), 7.61 (dd, 1H), 7.96 (dd, 1H) |
| Example 14 | Me | 1-Propenyl- | 95.0 ∼ 97.0 | (CDCℓ₃): 1.47 (d, 3H), 1.95 (dd, 3H), 2.11 (s, 6H), 4.27 (m, 1H), 5.01 (t, 1H), 5.33 (t, 1H), 5.96 (dd, 1H), 7.04 ∼ 7.45 (m, 1H), 7.55 (d, 1H) |

**Table 3**

| | Compounds Obtained | | | |
|---|---|---|---|---|
| | In Formula (IV) | | mp (°C) | ¹H-NMR (90MHz) δppm |
| | R¹ | R² | | |
| Example 15 | Me | Cyclohexyl- | 154.2 ∼ 155.1 | (CDCℓ₃): 1.47 (d, 3H), 1.1 ∼ 2.2 (m, 10H), 2.10 (s, 3H), 2.11 (s, 3H), 4.28 (m, 1H), 4.99 (t, 1H), 5.35 (t, 1H), 5.94 (dd, 1H), 7.60 (d, 1H) |
| Example 16 | Me | Benzyl- | 118.5 ∼ 119.8 | (CDCℓ₃): 1.46 (d, 3H), 2.09 (s, 3H), 2.10 (s, 3H), 4.21 (s, 2H), 4.28 (m, 1H), 4.99 (t, 1H), 5.35 (t, 1H), 5.95 (dd, 1H), 7.30 (s, 5H), 7.63 (d, 1H) |

### Example 17

1.10g of 3,4,5-trimethoxybenzoic acid was dissolved in 12mℓ of methylene chloride and 1.65g of pyridine, followed by addition of 0.60g of methanesulfonyl chloride at room temperature. After the mixture was stirred at room temperature for 2 hours, 1.32g of 5'-deoxy-2',3'-di-O-acetyl-5-fluorocytidine was added to the solution. After stirring at room temperature for 66 hours, the resulting solution was extracted with 10mℓ of water. The organic layer was separated and washed with 10mℓ of 4% sodium bicarbonate solution. After removal of the solvent under reduced pressure, the residue was recrystallized from ethyl acetate to obtain 1.27g (60.5%) of 5'-deoxy-2',3'-di-O-acetyl-5-fluoro-N⁴-(3,4,5-trimethoxybenzoyl)cytidine as a crystalline powder.

The results of instrumental analysis were identical with those obtained in Example 4.

### Example 18

12.9g of 5-fluorocytosine was suspended in 78mℓ of pyridine, followed by addition of 23.1g of 3,4,5-trimethoxybenzoyl chloride and stirring at 100°C for 5 hours. The reaction mixture was cooled down to room temperature and then poured into 310mℓ of water at room temperature over a period of 20 minutes. The precipitated crystals were collected by filtration to obtain 29.2g (90.4%) of 5-fluoro-N⁴-(3,4,5-trimethoxybenzoyl)cytosine.

The melting point of a product obtained by recrystallizing 14.6g of the above product from 600mℓ of methanol was 201.4 ∼ 202.2°C (decomposed).
¹H-NMR (90MHz, DMSO-d₆):
3.74 (s, 3H), 3.84 (s, 6H), 7.37 (s, 2H), 8.09 (d, J=5.9Hz, 1H)

6.47g of 5-fluoro-N⁴-(3,4,5-trimethoxybenzoyl)cytosine obtained by the above method were suspended in 10mℓ of toluene and reacted with 2.10g of hexamethyldisilazane at 100°C for 3 hours. The reaction solution was concentrated under reduced pressure. Then, 60mℓ of methylene chloride and 5.93g of 5-deoxy-1,2,3-tri-O-acetyl-β-D-ribofuranoside were added to the residue, followed by adding dropwise 6.25g of anhydrous stannic chloride while ice-cooling. This reaction solution was stirred at room temperature for further 30 minutes, followed by addition of 10.1g of sodium bicarbonate. 3.5 mℓ of water was then added at room temperature, over a period of 10 minutes. After stirring at room temperature for 3 hours, insoluble matters were filtered off, and the filtrate was washed with 10mℓ of 6% sodium bicarbonate solution. After the removal of the solvent under reduced pressure, the residue was recrystallized from 100mℓ of methanol to obtain 8.20g (78.3%) of 5'-deoxy-2',3'-di-O-acetyl-5-fluoro-N⁴-(3,4,5-trimethoxybenzoyl)cyti dine as a crystalline powder.

The results of instrumental analysis of the obtained compound was identical with those of Example 4.

### Example 19

5.55g of 5-fluoro-N⁴-(3,4,5-trimethoxybenzoyl)cytosine was suspended in 70mℓ of methylene chloride. After adding 5.09g of 5-deoxy-1,2,3-tri-O-acetyl-β-D-ribofuranoside to the suspension, 5.36g of anhydrous stannic chloride was added dropwise at room temperature over a period of 5 minutes. This reaction solution was stirred at room temperature for further 45 minutes. Thereafter, the reaction mixture was subjected to the same after-treatment as in Example 18 to obtain 6.17g (68.6%) of 5'-deoxy-2',3'-di-O-acetyl-5-fluoro-N⁴-(3,4,5-trimethoxybenzoyl)cytidine.

The results of instrumental analysis of the obtained compound were identical with those of Example 4.

### Example 20

35.5g of 5'-deoxy-2',3'-di-O-acetyl-5-fluoro-N⁴-(3,4,5-trimethoxybenzoyl)cytidine obtained according to the method of Example 4 was dissolved in 300mℓ of methylene chloride, to which 270mℓ of aqueous 1N-NaOH solution was dropwise added with stirring while ice-cooling. After stirring the solution for 30 minutes at the same temperature, 30mℓ of methanol was added to the reaction solution. After dropwise adding conc. hydrochloric acid to adjust the pH to 6, under ice-cooling the organic layer was separated, washed with 60mℓ of water and then concentrated under reduced pressure. The residue was crystallized from 150mℓ of ethyl acetate and filtered to obtain 25.4g (85.4%) of 5'-deoxy-5-fluoro-N⁴- (3,4,5-trimethoxybenzoyl)cytidine as crystals. The melting point of a product obtained by recrystallizing these crystals from ethyl acetate was 167.0 ∼ 168.4°C.
¹H-NMR (90MHz, DMSO-d₆):
1.34 (d, 3H), 3.75 (s, 3H), 3.85 (s, 6H), 5.08 (d, 1H), 5.45 (d, 1H), 5.73 (d, 1H), 7.36 (s, 2H), 8.22 (d, 1H)

### Example 21

52.3mg of 5'-deoxy-2'3'-di-O-acetyl-5-fluoro-N⁴-(3,4,5-trimethoxybenzoyl)cytidine was added to 0.4mℓ of N-NaOH, and the mixture was stirred at 26°C for 5 minutes. The progress of reaction was monitored by TLC. After completion of the reaction, the spot for the starting material had disappeared completely and that for 5'-deoxy-5-fluoro-N⁴-(3,4,5-trimethoxybenzoyl)cytidine alone was found. Methylene chloride was added to the reaction solution. Then, the pH of the solution was adjusted to pH 6 by dropwise addition of conc. hydrochloric acid. The organic layer was separated, washed with water and then concentrated under reduced pressure. The residue was recrystallized from ethyl acetate to obtain 5'-deoxy-5-fluoro-N⁴-(3,4,5-trimethoxybenzoyl)cytidine.

The results of instrumental analysis were identical with those of Example 20.

### Examples 22 ∼ 31

According to the method of Example 21, the reaction was carried out by selecting the compound of the formula (IV), kind of solvents, kind and amount of bases, reaction time and reaction temperature as given in Table 4 set forth below. Thereafter, the after-treatment was carried out in the same manner as in Example 21 to obtain 5'-deoxy-5-fluoro-N⁴-(3,4,5-trimethoxybenzoyl)cytidine.

In each example the spot for the starting material had disappeared completely and that for 5'-deoxy-5-fluoro-N⁴-(3,4,5-trimethoxybenzoyl)cytidine alone was found, after the reaction time given.

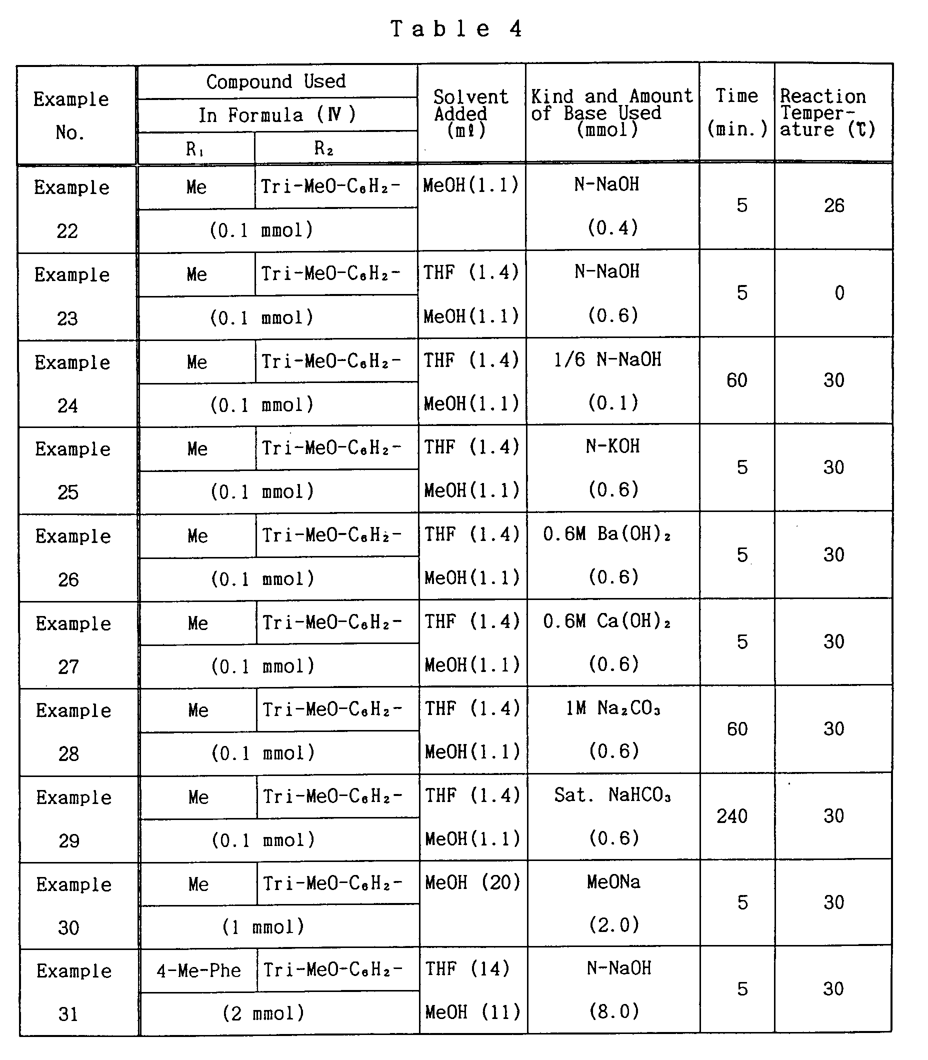

### Example 32

1.14g of 5'-deoxy-2',3'-di-O-acetyl-5-fluoro-N⁴-(palmitoyl)cytidine was dissolved in 14mℓ of THF and 11mℓ of methanol, followed by addition of 8mℓ of N-NaOH at 30°C and 5-minute stirring. The progress of the reaction was monitored by TLC. When the spot for the starting material had disappeared completely and that for the desired product alone was found, 10% hydrochloric acid was added to the reaction solution to adjust the pH to 5. After removal of the organic solvent under reduced pressure, the residue was extracted with 100mℓ of methylene chloride. The organic layer was separated, washed with water and concentrated under reduced pressure. After recrystallizing the residue from 7mℓ of methanol, the crystals were filtered off to obtain 0.64g (66%) of 5'-deoxy-5- fluoro-N⁴-palmitoyl-cytidine.

The results of instrumental analysis of the obtained compound were as follows.
Melting point: 93.0 ∼95.0°C.
¹H-NMR (90MHz, DMSO-d₆):
0.86 (t, 3H), 1.24 (s, methylene), 1.33 (d, 3H), 3.5 ∼ 4.15 (m, 3H), 5.04 (d, 1H), 5.42 (d, 1H), 5.68 (dd, 1H), 8.08 (d, 1H)

### Example 33

0.98g of 5'-deoxy-2',3'-di-O-acetyl-5-fluoro-N⁴-(4-chlorobenzoyl)cytidine was dissolved in 14mℓ of THF and 11mℓ of methanol, followed by addition of 8mℓ of N-NaOH at 30°C and 5-minute stirring. When the spot for the starting material had disappeared completely and that for the desired product alone was found the reaction solution was treated in the same manner as in Example 32, and the obtained residue was recrystallized from ethyl acetate to obtain 0.40g (49.8%) of N⁴-(4-chlorobenzoyl)-5'-deoxy-5-fluorocytidine.

The results of instrumental analysis of the obtained compound were given below.
Melting point: 142.3 ∼145.6°C.
¹H-NMR (90MHz, DMSO-d₆):
1.32 (d, 3H), 3.5 ∼ 4.2 (m, 3H), 5.08 (d, 1H), 5.42 (d, 1H), 5.71 (dd, 1H), 7.58 (d, 2H), 8.02 (d, 1H), 8.02 (d, 2H)

### Example 34

To a solution of 0.93g of 5'-deoxy-2',3'-di-O-acetyl-5-fluoro-N⁴-(2-methoxybenzoyl)cytidine in 14mℓ of THF and 11mℓ of methanol was added 8mℓ of N-NaOH at 30°C, and the mixture was stirred for 5 minutes. When the spot for the starting material had disappeared completely and that for the aimed product alone was found the reaction solution was treated in the same manner as in Example 32, and the obtained residue was recrystallized from methanol to give 0.40g (52.5%) of 5'-deoxy-5-fluoro-N⁴-(2-methoxybenzoyl)cytidine.

The results of instrumental analysis of the obtained compound were as follows.
Melting Point: 196.8 ∼ 197.9°C (Decomposed).
¹H-NMR (90MHz, DMSO-d₆):
1.34 (d, 3H), 3.93 (s, 3H), 3.5 ∼ 4.3 (m, 3H), 5.05 (d, 1H), 5.45 (d, 1H), 5.70 (dd, 1H), 7.1 ∼ 7.8 (m, 4H), 8.15 (d, 1H)

### Example 35

To a solution of 447mg of 5'-deoxy-2',3'-di-O-acetyl-5-fluoro-N⁴-(3-methylbenzoyl)cytidine in 25mℓ of methylene chloride was added 8mℓ of 0.5N-NaOH at 14°C, and the mixture was stirred for 5 minutes. When the spot for the starting material had completely disappeared and that for the aimed product alone was found 10% hydrochloric acid was added to the reaction solution to adjust the pH to 5, and the resulting solution was extracted with 5mℓ of methanol. After extracting the aqueous layer with additional 10mℓ of methylene chloride, the organic layers were combined and washed with water(10mℓ). After distilling the organic solvent under reduced pressure, the residue was recrystallized from 4mℓ of ethanol to obtain 301mg (82.9%) of 5'-deoxy-5-fluoro-N⁴-(3-methylbenzoyl)cytidine.
Melting Point: 146.5 ∼ 147.8°C

### Examples 36 ∼ 44

In accordance with the method of Example 35, the compounds (IV) given in Table 5 were dissolved in a suitable quantity of methylene chloride and then subjected to selective deacylation in the presence of 0.5N-NaOH as was equivalent to 4 times the mole of each corresponding compound. Thereafter, the after-treatments were carried out in the same manner as in Example 35 to obtain the aimed compounds.

Reaction conditions thereof and Yields of the obtained compounds were as given in Table 5, and the physicochemical properties of the compounds were confirmed by instrument analyses such as melting point, NMR, etc.

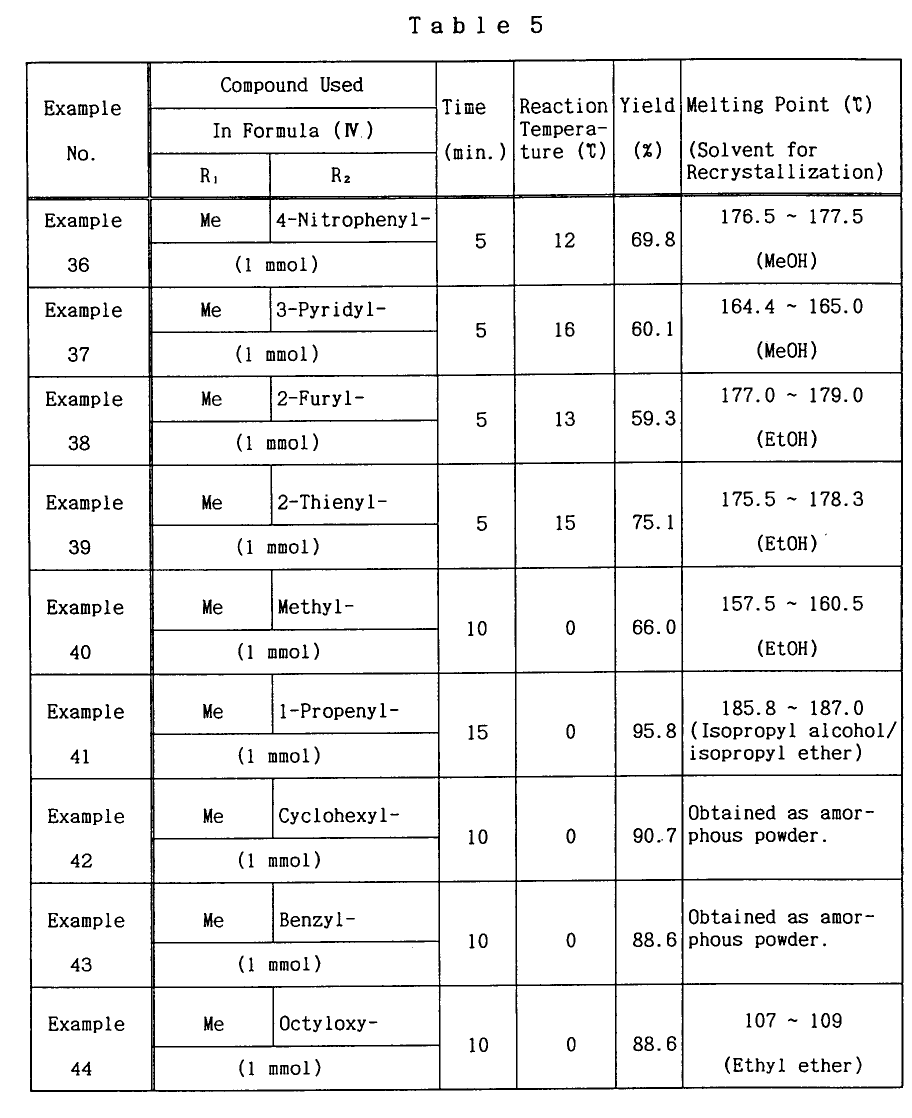

### Example 45

52.3mg of 5'-deoxy-2',3'-di-O-acetyl-5-fluoro-N⁴-(3,4,5-trimethoxybenzoyl)cytidine was suspended in 0.44mℓ of water. After dropwise adding 0.36mℓ of 10% tetramethylammoniumhydroxide solution to the suspension with stirring at 26°C, the mixture was stirred at the same temperature for 5 minutes. When the spot for the starting material had completely disappeared and that for the aimed product alone was found, the after-treatment was carried out in the same manner as in Example 21 to obtain 5'-deoxy-5-fluoro-N⁴-(3,4,5-trimethoxybenzoyl)cytidine.

The results of instrumental analysis were identical with those of Example 20.

### Example 46

52.3mg of 5'-deoxy-2',3'-di-O-acetyl-5-fluoro-N⁴-(3,4,5-trimethoxybenzoyl)cytidine was suspended in 0.74mℓ of water. After dropwise adding 60µℓ of DBU to the suspension with stirring at 26°C, the mixture was stirred at the same temperature for 5 minutes. When the spot for the starting material had completely disappeared and that for the aimed product alone was found the after-treatment was carried out in the same manner as in Example 21 to obtain 5'-deoxy-5-fluoro-N⁴-(3,4,5-trimethoxybenzoyl)cytidine.

The results of instrumental analysis were identical with those of Example 20.

### Example 47

52.3mg of 5'-deoxy-2',3'-di-O-acetyl-5-fluoro-N⁴-(3,4,5-trimethoxybenzoyl)cytidine was suspended in 0.74mℓ of water. After dropwise adding 55.5µℓ of triethylamine to the suspension with stirring at 27°C, the mixture was stirred at the same temperature for 3 hours. When the spot for the starting material had completely disappeared and that for the aimed product alone was found the after-treatment was carried out in the same manner as in Example 21 to obtain 5'-deoxy-5-fluoro-N⁴-(3,4,5-trimethoxybenzoyl)cytidine.

The results of instrumental analysis were identical with those of Example 20.

### Example 48

25.8g of 5-fluorocytosine was suspended in 100mℓ of toluene and 21g of hexamethyldisilazane, and the suspension was heated to react at 110°C for 3 hours. After concentrating the reaction mixture under reduced pressure, 330mℓ of methylene chloride and then 59.3g of 5-deoxy-1,2,3-tri-O-acetyl-β-D-ribofranoside were added to the residue. To the ice-cooled solution was added dropwise 62.5g of anhydrous stannic chloride over a period of in 10 minutes. After the mixture was stirred at room temperature for further 2 hours, 101g of sodium bicarbonate was added thereto at room temperature, followed by dropwise addition of 35mℓ of water over a period of 20 minutes. The mixture was stirred at room temperature for 3 hours, and then the insoluble matters were filtered off. The filtrate was washed with 100mℓ of 4% aqueous sodium bicarbonate solution and dried (Na₂SO₄). Then, 23g of pyridine was added to the above solution, into which 56g of 3,4,5-trimethoxybenzoyl chloride was added at room temperature. After stirring the mixture at room temperature for overnight, the reaction mixture was partitioned between 250mℓ of methylene chloride and 125mℓ of water. The organic layer was separated and then washed with 125mℓ of 4% aqueous sodium bicarbonate solution.

Into the organic layer obtained above was added dropwise 732mℓ of N-NaOH solution with stirring while ice-cooling. After stirring for 30 minutes, 200mℓ of methylene chloride and 70mℓ of methanol were added to the reaction mixture. After adjusting the resulting solution to pH 6 with conc. hydrochloric acid, the organic layer was separated, washed with 160mℓ of water and concentrated under reduced pressure. The residue was crystallized from 370mℓ of ethyl acetate, and the crystals were filtered. These crystals were recrystallized from 1,150mℓ of ethyl acetate to obtain 56.0g (63.8%) of 5'-deoxy-5-fluoro-N⁴-(3,4,5-trimethoxybenzoyl)cytidine as crystals.

The results of instrumental analysis of the obtained compound were identical with those of Example 20.

### Example 49

To a suspension of 12.9g of fluorocytosine in 78mℓ of pyridine was added 23.1g of 3,4,5-trimethoxybenzoyl chloride, and the mixture was reacted at 100°C for 5 hours. After the completion of reaction, the reaction mixture was poured into 300mℓ of water at room temperature with stirring. After stirring the resulting mixture for additional 3 hours, the precipitated crystals were collected by filtration, washed with water and then dried to obtain 29.2g of 5-fluoro-N⁴-(3,4,5-trimethoxybenzoyl)cytosine.

The crystals obtained above were suspended in 46mℓ of toluene and 9.5g of hexamethyldisilazane, and the mixture was heated to react at 110°C for 3 hours. After evaporation of the reaction mixture under reduced pressure. 270mℓ of methylene chloride and 26.8g of 5-deoxy-1,2,3-tri-O-acetyl-β-D-ribofuranoside was added to the residue, to which 28.2g of anhydrous stannic chloride was added dropwise over a period of 5 minutes while ice-cooling. After stirring this solution at room temperature for additional 1 hour, 45.5g of sodium bicarbonate was added, followed by further of 16mℓ of water over a period of 10 minutes. After stirring the mixture for 3 hours, insoluble matters were filtered off, and the filtrate was washed with 45mℓ of 6% sodium bicarbonate solution. To the organic layer obtained according to the above method, 292mℓ of N-NaOH was added dropwise with stirring while ice-cooling. After stirring for 30 minutes at the same temperature, 50mℓ of methylene chloride and 30mℓ of methanol were added to the mixture. After adjusting the mixture to pH 6 with concentrated hydrochloric acid, the organic layer was separated, washed with water and then concentrated under reduced pressure. The residue was crystallized from 150mℓ of ethyl acetate, and the crystals were filtered. The filtered crystals were recrystallized from 480mℓ of ethyl acetate to obtain 23.2g (52.8%) of 5'-deoxy-5-fluoro-N⁴-(3,4,5-trimethoxybenzoyl)cytidine as crystals.

The results of instrumental results of the obtained compound were identical with those of Example 20.

## Claims

1. A process for producing N⁴-acyl-5'-deoxy-5-fluorocytidine derivatives represented by the general formula (V). 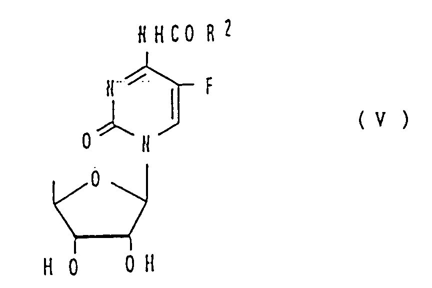 wherein R² is an alkyl radical, a cycloalkyl radical, an alkenyl radical, an aralkyl radical, an aryl radical or an alkoxy radical,
characterized by reacting 5-fluorocytosine with a compound represented by the general formula (II), 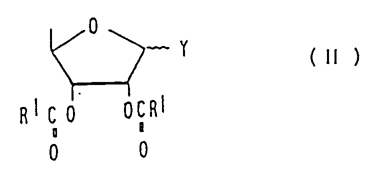 wherein R¹ is a lower alkyl radical, an aryl radical or an aryl radical which may have (a) substituent(s) and Y is a halogen atom, an acyloxy radical or an alkoxy radical,
to give a compound represented by the general formula (III), 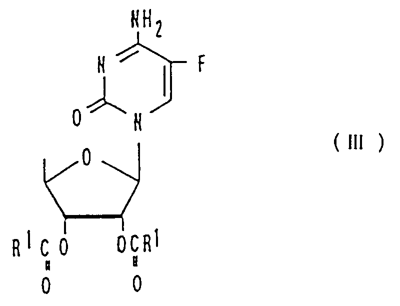 wherein R¹ is the same as defined above,
reacting this compound with an acylating agent to introduce an R²CO radical into its amino radical to produce a compound represented by the general formula (IV), 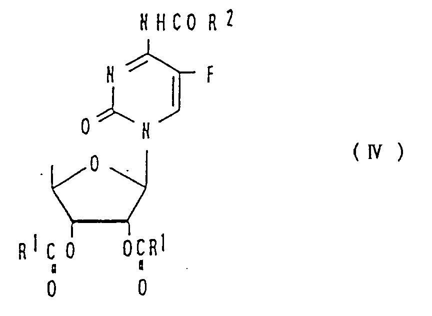 wherein R¹ and R² are the same as defined above,
and selectively deacylating only R¹CO radicals of this compound by treatment with a base in a solvent.

2. A process for producing N⁴-acyl-5'-deoxy-5-fluorocytidine derivatives represented by the general formula (V), 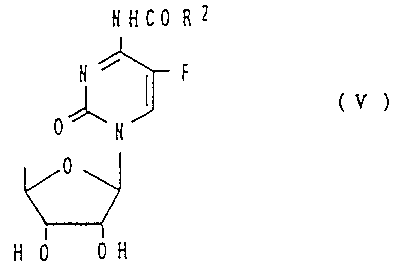 wherein R² is an alkyl radical, a cycloalkyl radical, an alkenyl radical, an aralkyl radical, an aryl radical or an alkoxy radical,
characterized by reacting 5-fluorocytosine with an acylating agent to introduce an R²CO radical into its amino radical to produce a compound represented by the general formula (VI), 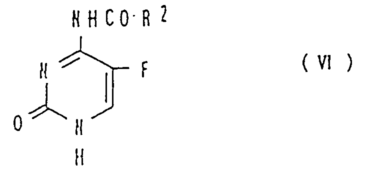 wherein R² is the same as defined above,
reacting said compound with a compound represented by the general formula (II), 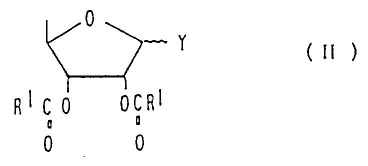 wherein R¹ is a lower alkyl radical, an aryl radical or an aryl radical which may have (a) substituent(s) and Y is a halogen atom, an acyloxy radical or an alkoxy radical,
to produce a compound represented by the general formula (IV), 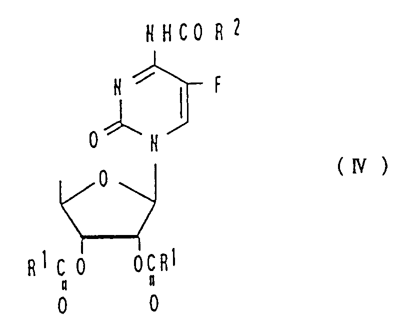 wherein R¹ and R² are the same as defined above,
and selectively deacylating only an R¹CO radical of this compound by treatment with a base in a solvent.

3. A process for producing N⁴-acyl-5'-deoxy-5-fluorocytidine derivatives represented by the general formula (V), 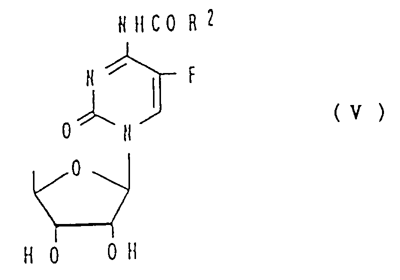 wherein R² is an alkyl radical, a cycloalkyl radical, an alkenyl radical, and aralkyl radical, an aryl radical or an alkoxy radical,
which comprises treating a compound represented by the general formula (IV), 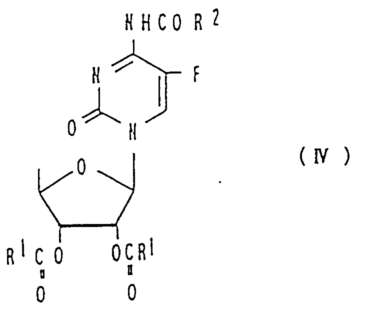 wherein R¹ is a lower alkyl radical, an aryl radical or an aryl radical which may have(a)substituent(s) and R² are the same as above,
in a solvent in the presence of a base substance to selectively deacylate only R¹CO radicals of this compound.

## Patentansprüche

1. Verfahren zur Herstellung von N⁴-Acyl-5'-deoxy-5-fluorocytidin-derivaten der allgemeinen Formel (V) 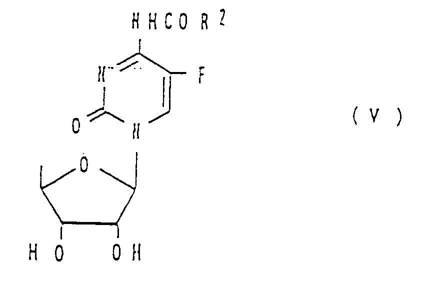 worin R² ein Alkylrest, ein Cycloalkylrest, ein Alkenylrest, ein Aralkylrest, ein Arylrest oder ein Alkoxyrest ist,
dadurch gekennzeichnet, dass man 5-Fluorocytosin mit einer Verbindung der allgemeinen Formel (II) 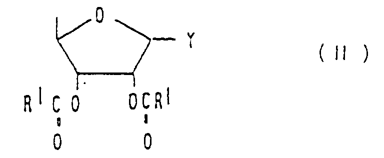 worin R¹ ein niederer Alkylrest, ein Arylrest oder ein Arylrest der Substituenten haben kann, und Y ein Halogenatom, ein Acyloxyrest oder ein Alkoxyrest ist,
zu einer Verbindung der allgemeinen Formel (III) 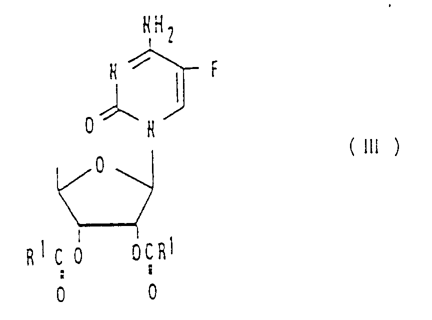 worin R¹ dasselbe wie oben definiert ist
umsetzt, diese Verbindung mit einem Acylierungsmittel umsetzt um einen R²CO Rest in seinen Aminorest einzuführen und eine Verbindung der allgemeinen Formel (IV) herzustellen 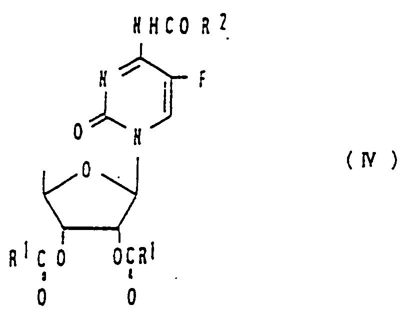 worin R¹ und R² dasselbe wie oben definiert sind
und selektiv nur R¹CO Reste dieser Verbindung durch Behandlung mit einer Base in einem Lösungsmittel deacyliert.

2. Verfahren zur Herstellung von N⁴-Acyl-5'-deoxy-5-fluorocytidin-derivaten der allgemeinen Formel (V) 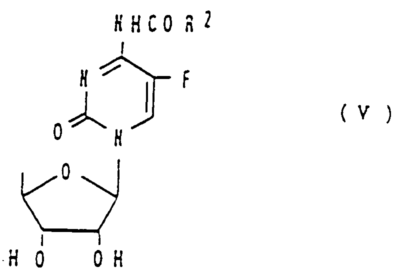 worin R² ein Alkylrest, ein Cycloalkylrest, ein Alkenylrest, ein Aralkylrest, ein Arylrest oder ein Alkoxyrest ist,
dadurch gekennzeichnet, dass man 5-Fluorocytosin mit einem Acylierungsmittel umsetzt um einen R²CO Rest in seinen Aminorest einzuführen und eine Verbindung der allgemeinen Formel (VI) 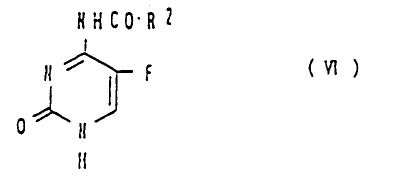 worin R² dasselbe wie oben definiert ist
zu erhalten,
die genannte Verbindung mit einer Verbindung der allgemeinen Formel (II) 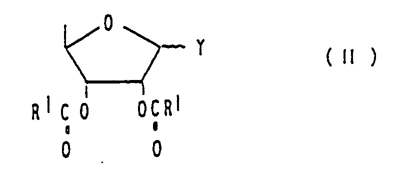 worin R¹ ein niederer Alkylrest, ein Arylrest oder ein Arylrest der Substituenten haben kann und Y ein Halogenatom, ein Acyloxyrest oder ein Alkoxyrest ist,
zu einer Verbindung der allgemeinen Formel (IV) 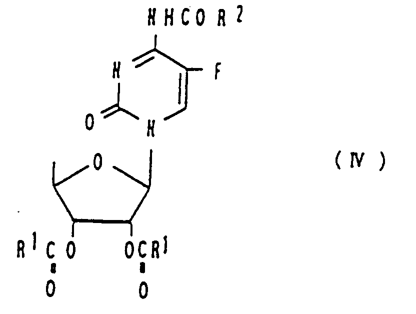 worin R¹ und R² dasselbe wie oben definiert sind
umsetzt und selektiv nur einen R¹CO Rest dieser Verbindung durch Behandlung einer Base in einem Lösungsmittel deacyliert.

3. Verfahren zur Herstellung von N⁴-Acyl-5'-deoxy-5-fluorocytidin derivaten der allgemeinen Formel (V) 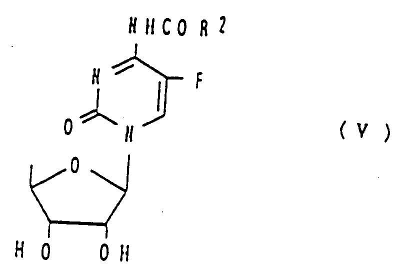 worin R² ein Alkylrest, ein Cycloalkylrest, ein Alkenylrest, ein Aralkylrest, ein Arylrest oder ein Alkoxyrest ist,
dadurch gekennzeichnet, dass man eine Verbindung der allgemeinen Formel (IV) 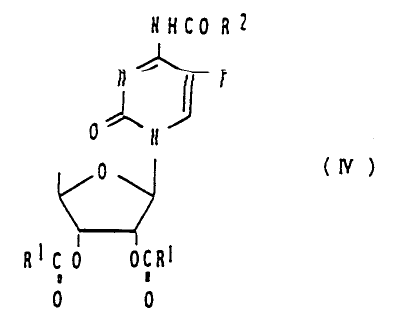 worin R¹ ein niederer Alkylrest, ein Arylrest oder ein Arylrest der Substituenten haben kann und R² dasselbe wie oben ist,
in einem Lösungsmittel in Gegenwart einer basischen Substanz behandelt und selektiv nur R¹CO Reste dieser Verbindung deacyliert.

## Revendications

1. Procédé de production de dérivés de N⁴-acyl-5'-désoxy-5-fluoro-cytidine représentés par la formule générale (V) : 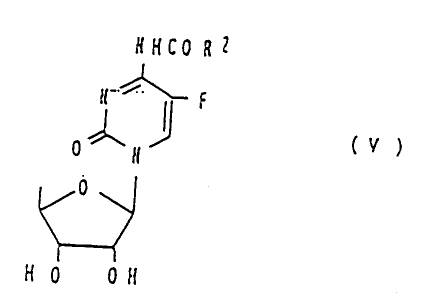 où R² est un radical alkyle, un radical cycloalkyle, un radical alcényle, un radical aralkyle, un radical aryle ou un radical alcoxy,
caractérisé en ce qu'on fait réagir la 5-fluorocytosine avec un composé représenté par la formule générale (II) : 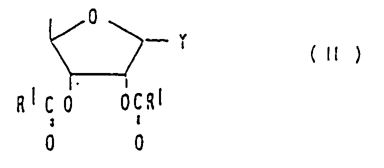 où R' est un radical alkyle inférieur, un radical aryle ou un radical aryle qui peut avoir un ou plusieurs substituants et Y est un atome d'halogène, un radical acyloxy ou un radical alcoxy, pour donner un composé représenté par la formule générale (III) : 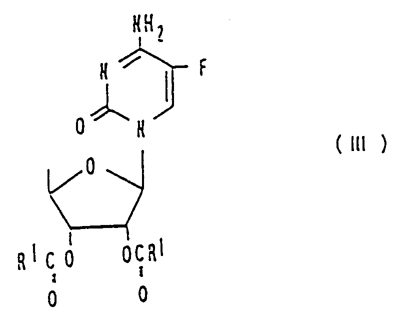 où R¹ est tel que défini ci-dessus,
on fait réagir ce composé avec un agent d'acylation pour introduire un radical R²CO dans son radical amino pour produire un composé représenté par la formule générale (IV) : 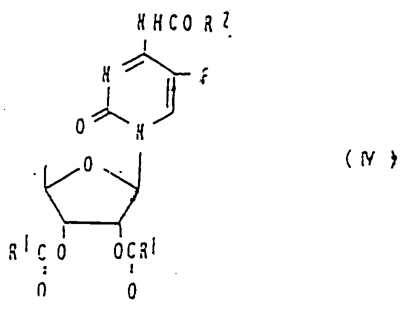 où R¹ et R² sont tels que définis ci-dessus,
et on désacyle sélectivement seulement les radicaux R¹CO de ce composé par traitement avec une base dans un solvant.

2. Procédé de production de dérivés de N⁴-acyl-5'-désoxy-5-fluoro-cytidine représentés par la formule générale (V) : 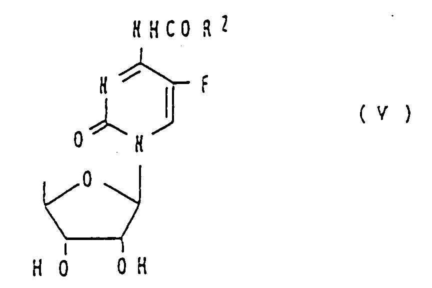 où R² est un radical alkyle, un radical cycloalkyle, un radical alcényle, un radical aralkyle, un radical aryle ou un radical alcoxy,
caractérisé en ce qu'on fait réagir la 5-fluoro-cytosine avec un agent d'acylation pour introduire un radical R²CO dans son radical amino pour produire un composé représenté par la formule générale (VI) : 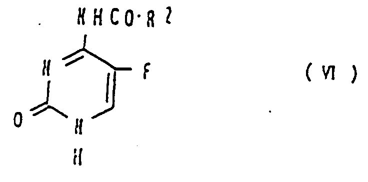 où R² est tel que défini ci-dessus,
on fait réagir ledit composé avec un composé représenté par la formule générale (II) : 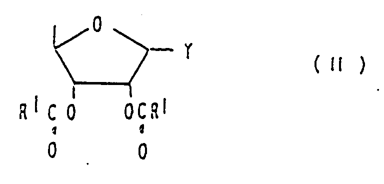 où R¹ est un radical alkyle inférieur, un radical aryle ou un radical aryle qui peut avoir un ou plusieurs substituants et Y est un atome d'halogène, un radical acyloxy ou un radical alcoxy,
pour produire un composé représenté par la formule générale (IV) : 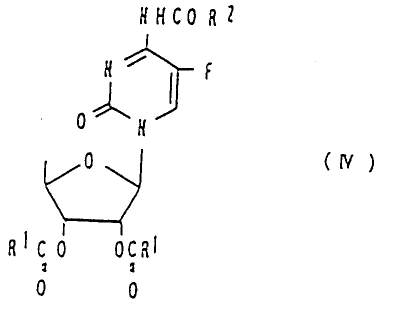 où R¹ et R² sont tels que définis ci-dessus, et désacyler sélectivement seulement un radical R¹CO de ce composé, par traitement avec une base dans un solvant.

3. Procédé de production de dérivés N⁴-acyl-5'-désoxy-5-fluoro-cytidine représentés par la formule générale (V) : 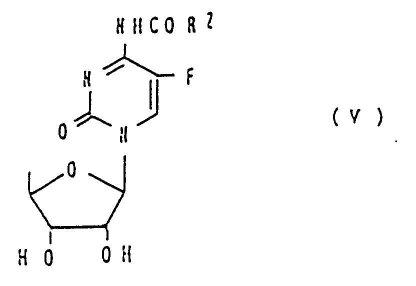 où R² est un radical alkyle, un radical cycloalkyle, un radical alcényle et un radical aralkyle, un radical aryle ou un radical alcoxy,
qui comprend de traiter un composé représenté par la formule générale (IV) : 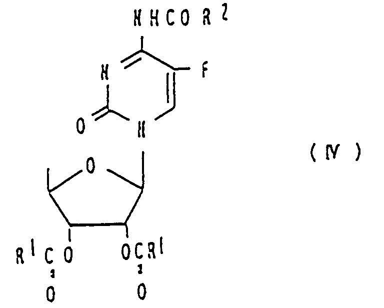 où R¹ est un radical alkyle inférieur, un radical aryle ou un radical aryle qui peut avoir un ou plusieurs substituants, et R² est tel que ci-dessus,
dans un solvant en présence d'une substance basique pour désacyler sélectivement seulement les radicaux R¹CO de ce composé.
